# EUROPEAN PATENT SPECIFICATION

(11) **EP 1 192 185 B1**
(45) Date of publication and mention of the grant of the patent: **21.06.2006**
(21) Application number: 00941233.9
(22) Date of filing: 07.06.2000
(51) Int. Cl.: C07K 16/00

(54) **Apo-2L RECEPTOR AGONIST AND CPT-11 SYNERGISM**
APO-2L REZEPTOR-AGONIST UND CPT-11 SYNERGIE-EFFEKT
SYNERGIE D'AGONISTE DES RECEPTEURS DE LIGAND APO-2 (APO-2L) ET DE CPT-11

(30) Priority: 09.06.1999 US 138240 P
(43) Date of publication of application: 03.04.2002
(60) Divisional application: 06003358.6
(73) Proprietor: GENENTECH, INC., South San Francisco, CA 94080-4990 (US)
(72) Inventor: ASHKENAZI, Avi, J., San Mateo, CA 94402 (US); BENYUNES, Mark, C., San Francisco, CA 94117 (US); SCHWALL, Ralph, H., Pacifica, CA 94044 (US)
(74) Representative: Kiddle, Simon John
(86) International application number: PCT/US2000/015512
(87) International publication number: WO 2000/075191

(56) References cited:
- WO-A-97/25428
- WO-A-99/37684
- SCHILLER, JOAN H.: "Topotecan in small-cell lung cancer" SEMIN. ONCOL. (1997), 24(6, SUPPL. 20), S20/27-S20/33 , XP000973356
- PAN, GUOHUA ET AL: "The receptor for the cytotoxic ligand TRAIL" SCIENCE (WASHINGTON, D. C.) (1997), 276(5309), 111 - 113 , XP002158405 cited in the application
- ASHKENAZI A ET AL: "Safety and antitumor activity of recombinant soluble Apo2 ligand." JOURNAL OF CLINICAL INVESTIGATION, (1999 JUL) 104 (2) 155-62. , XP000973369

## Description

### FIELD OF THE INVENTION

This invention relates generally to methods of inducing apoptosis in mammalian cells. In particular, it pertains to the use of Apo-2L receptor agonists and CPT-11 to synergistically induce apoptosis in mammalian cells. Various Apo-2L receptor agonists contemplated by the invention include the ligand known as Apo-2 ligand or TRAIL, as well as agonist antibodies directed to one or more Apo-2L receptors.

### BACKGROUND OF THE INVENTION

Various molecules, such as tumor necrosis factor-α ("TNF-α"), tumor necrosis factor-β ("TNF-β" or "lymphotoxin-α") , lymphotoxin-β ("LT-β"), CD30 ligand, CD27 ligand, CD40 ligand, OX-40 ligand, 4-1BB ligand, Apo-1 ligand (also referred to as Fas ligand or CD95 ligand), Apo-2 ligand (also referred to as TRAIL), Apo-3 ligand (also referred to as TWEAK), osteoprotegerin (OPG), APRIL, RANK ligand (also referred to as TRANCE), and TALL-1 (also referred to as BlyS, BAFF or THANK) have been identified as members of the tumor necrosis factor ("TNF") family of cytokines [See, e.g., Gruss and Dower, Blood, 85:3378-3404 (1995); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992), WO 97/01633 published January 16, 1997; WO 97/25428 published July 17, 1997; Marsters et al., Curr. Biol., 8:525-528 (1998); Simonet et al., Cell, 89:309-319 (1997); Chicheportiche et al., Biol. Chem., 272:32401-32410 (1997); Hahne et al., J. Exp. Med., 188:1185-1190 (1998); WO98/28426 published July 2, 1998; WO98/46751 published October 22, 1998; WO/98/18921 published May 7, 1998; Moore et al., Science, 285:260-263 (1999); Shu et al., J. Leukocyte Biol., 65:680 (1999); Schneider et al., J. Exp. Med., 189:1747-1756 (1999); Mukhopadhyay et al., J. Biol. Chem., 274:15978-15981 (1999)]. Among these molecules, TNF-α, TNF-β, CD30 ligand, 4-1BB ligand, Apo-1 ligand, Apo-2 ligand (Apo2L/TRAIL) and Apo-3 ligand (TWEAK) have been reported to be involved in apoptotic cell death. Both TNF-α and TNF-β have been reported to induce apoptotic death in susceptible tumor cells [Schmid et al., Proc. Natl. Acad. Sci., 83:1881 (1986); Dealtry et al., Eur. J. Immunol., 17:689 (1987)].

Recently, additional molecules believed to be members of the TNF cytokine family were identified and reported to be involved in apoptosis. For instance, in Pitti et al., J. Biol. Chem., 271:12687-12690 (1996), a molecule referred to as Apo-2 ligand is described. See also, WO 97/25428 published July 17, 1997. The full length human Apo-2 ligand is reported to be a 281 amino acid polypeptide that induces apoptosis in various mammalian cells. Other investigators have described related polypeptides referred to as TRAIL [Wiley et al., Immunity, 3:673-682 (1995); WO 97/01633 published January 16, 1997] and AGP-1 [WO 97/46686 published December 11, 1997].

Various molecules in the TNF family also have purported role(s) in the function or development of the immune system [Gruss et al., Blood, 85:3378 (1995)]. Zheng et al. have reported that TNF-α is involved in post-stimulation apoptosis of CD8-positive T cells [Zheng et al., Nature, 377:348-351 (1995)]. Other investigators have reported that CD30 ligand may be involved in deletion of self-reactive T cells in the thymus [Amakawa et al., Cold Spring Harbor Laboratory Symposium on Programmed Cell Death, Abstr. No. 10, (1995)]. CD40 ligand activates many functions of B cells, including proliferation, immunoglobulin secretion, and survival [Renshaw et al., J. Exp. Med., 180:1889 (1994)]. Another recently identified TNF family cytokine, TALL-1 (BlyS), has been reported, under certain conditions, to induce B cell proliferation and immunoglobulin secretion. [Moore et al., supra; Schneider et al., supra; Mackay et al., J. Exp. Med., 190:1697 (1999)].

Mutations in the mouse Fas/Apo-1 receptor or ligand genes (called *lpr* and gld, respectively) have been associated with some autoimmune disorders, indicating that Apo-1 ligand may play a role in regulating the clonal deletion of self-reactive lymphocytes in the periphery [Krammer et al., Curr. Op. Immunol., 6:279-289 (1994); Nagata et al., Science, 267:1449-1456 (1995)]. Apo-1 ligand is also reported to induce post-stimulation apoptosis in CD4-positive T lymphocytes and in B lymphocytes, and may be involved in the elimination of activated lymphocytes when their function is no longer needed [Krammer et al., supra; Nagata et al., supra]. Agonist mouse monoclonal antibodies specifically binding to the Apo-1 receptor have been reported to exhibit cell killing activity that is comparable to or similar to that of TNF-α [Yonehara et al., J. Exp. Med., 169:1747-1756 (1989)].

Induction of various cellular responses mediated by such TNF family cytokines is believed to be initiated by their binding to specific cell receptors. Previously, two distinct TNF receptors of approximately 55-kDa (TNFR1) and 75-kDa (TNFR2) were identified [Hohman et al., J. Biol. Chem., 264:14927-14934 (1989); Brockhaus et al., Proc. Natl. Acad. Sci., 87:3127-3131 (1990); EP 417,563, published March 20, 1991; Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)]. Those TNFRs were found to share the typical structure of cell surface receptors including extracellular, transmembrane and intracellular regions. The extracellular portions of both receptors were found naturally also as soluble TNF-binding proteins [Nophar, Y. et al., EMBO J., 9:3269 (1990); and Kohno, T. et al., Proc. Natl. Acad. Sci. U.S.A., 87:8331 (1990); Hale et al., J. Cell. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. [Schall et al., supra; Loetscher et al., supra; Smith et al., supra; Nophar et al., supra; Kohno et al., supra; Banner et al., Cell, 73:431-435 (1993)]. A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallet et al., EMBO J., 9:1063 (1990)] and the Fas antigen [Yonehara et al., supra and Itoh et al., Cell, 66:233-243 (1991)]. CRDs are also found in the soluble TNFR (sTNFR)-like T2 proteins of the Shope and myxoma poxviruses [Upton et al., Virology, 160:20-29 (1987); Smith et al., Biochem. Biophys. Res. Commun., 176:335 (1991); Upton et al., Virology, 184:370 (1991)]. Optimal alignment of these sequences indicates that the positions of the cysteine residues are well conserved. These receptors are sometimes collectively referred to as members of the TNF/NGF receptor superfamily.

The TNF family ligands identified to date, with the exception of lymphotoxin-α, are type II transmembrane proteins, whose C-terminus is extracellular. In contrast, most receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

More recently, other members of the TNFR family have been identified. In von Bulow et al., Science, 278:138-141 (1997), investigators describe a plasma membrane receptor referred to as Transmembrane Activator and CAML-Interactor or "TACI". The TACI receptor is reported to contain a cysteine-rich motif characteristic of the TNFR family. In an *in vitro* assay, cross linking of TACI on the surface of transfected Jurkat cells with TACI-specific antibodies led to activation of NF-KB. [see also, WO 98/39361 published September 18, 1998].

Laabi et al., EMBO J., 11:3897-3904 (1992) reported identifying a new gene called "BCM" whose expression was found to coincide with B cell terminal maturation. The open reading frame of the BCM normal cDNA predicted a 184 amino acid long polypeptide with a single transmembrane domain. These investigators later termed this gene "BCMA." [Laabi et al., Nucleic Acids Res., 22:1147-1154 (1994)]. BCMA mRNA expression was reported to be absent in human malignant B cell lines which represent the pro-B lymphocyte stage, and thus, is believed to be linked to the stage of differentiation of lymphocytes [Gras et al., Int. Immunology, 7:1093-1106 (1995)]. In Madry et al., Int. Immunology, 10:1693-1702 (1998), the cloning of murine BCMA cDNA was described. The murine BCMA cDNA is reported to encode a 185 amino acid long polypeptide having 62% identity to the human BCMA polypeptide. Alignment of the murine and human BCMA protein sequences revealed a conserved motif of six cysteines in the N-terminal region, suggesting that the BCMA protein belongs to the TNFR superfamily [Madry et al., supra].

In Marsters et al., Curr. Biol., 6:750 (1996), investigators describe a full length native sequence human polypeptide, called Apo-3, which exhibits similarity to the TNFR family in its extracellular cysteine-rich repeats and resembles TNFR1 and CD95 in that it contains a cytoplasmic death domain sequence [see also Marsters et al., Curr. Biol., 6:1669 (1996)]. Apo-3 has also been referred to by other investigators as DR3, wsl-1, TRAMP, and LARD [Chinnaiyan et al., Science, 274:990 (1996); Kitson et al., Nature, 384:372 (1996); Bodmer et al., Immunity, 6:79 (1997); Screaton et al., Proc. Natl. Acad. Sci., 94:4615-4619 (1997)].

Pan et al. have disclosed another TNF receptor family member referred to as "DR4" [Pan et al., Science, 276:111-113 (1997); see also W098/32856 published July 30, 1998]. The DR4 was reported to contain a cytoplasmic death domain capable of engaging the cell suicide apparatus. Pan et al. disclose that DR4 is believed to be a receptor for the ligand known as Apo2L/TRAIL.

In Sheridan et al., Science, 277:818-821 (1997) and Pan et al., Science, 277:815-818 (1997), another molecule believed to be a receptor for Apo2L/TRAIL is described [see also, WO98/51793 published November 19, 1998; WO98/41629 published September 24, 1998]. That molecule is referred to as DR5 (it has also been alternatively referred to as Apo-2; TRAIL-R, TR6, Tango-63, hAP08, TRICK2 or KILLER [Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/4664 published October 22, 1998; WO99/02653 published January 21, 1999; WO99/09165 published February 25, 1999; WO99/1179 published March 11, 1999]. Like DR4, DR5 is reported to contain a cytoplasmic death domain and be capable of signaling apoptosis. The crystal structure of the complex formed between Apo-2L/TRAIL and DR5 is described in Hymowitz et al., Molecular Cell, 4:563-571 (1999).

Yet another death domain-containing receptor, DR6, was recently identified [Pan et al., FEBS Letters, 431:351-356 (1998)]. Aside from containing four putative extracellular cysteine rich domains and a cytoplasmic death domain, DR6 is believed to contain a putative leucine-zipper sequence that overlaps with a proline-rich motif in the cytoplasmic region. The proline-rich motif resembles sequences that bind to src-homology-3 domains, which are found in many intracellular signal-transducing molecules.

A further group of recently identified receptors are referred to as "decoy receptors," which are believed to function as inhibitors, rather than transducers of signaling. This group includes DCR1 (also referred to as TRID, LIT or TRAIL-R3) [Pan et al., Science, 276:111-113 (1997); Sheridan et al., Science, 277:818-821 (1997); McFarlane et al., J. Biol. Chem., 272:25417-25420 (1997); Schneider et al., FEBS Letters, 416:329-334 (1997); Degli-Esposti et al., J. Exp. Med., 186:1165-1170 (1997); and Mongkolsapaya et al., J. Immunol., 160:3-6 (1998)] and DCR2 (also called TRUNDD or TRAIL-R4) [Marsters et al., Curr. Biol., 7:1003-1006 (1997); Pan et al., FEBS Letters, 424:41-45 (1998); Degli-Esposti et al., Immunity, 7:813-820 (1997)], both cell surface molecules, as well as OPG [Simonet et al., supra; Emery et al., infra] and DCR3 [Pitti et al., Nature, 396:699-703 (1998)], both of which are secreted, soluble proteins.

Additional newly identified members of the TNFR family include CAR1, HVEM, GITR, ZTNFR-5, NTR-1, and TNFL1 [Brojatsch et al., Cell, 87:845-855 (1996); Montgomery et al., Cell, 87:427-436 (1996); Marsters et al., J. Biol. Chem., 272:14029-14032 (1997); Nocentini et al., Proc. Natl. Acad. Sci. USA 94:6216-6221 (1997); Emery et al., J. Biol. Chem., 273:14363-14367 (1998); WO99/04001 published January 28, 1999; WO99/07738 published February 18, 1999; WO99/3398 published July 8, 1999].

As reviewed recently by Tewari et al., TNFR1, TNFR2 and CD40 modulate the expression of proinflammatory and costimulatory cytokines, cytokine receptors, and cell adhesion molecules through activation of the transcription factor, NF-κB [Tewari et al., Curr. Op. Genet. Develop., 6:39-44 (1996)]. NF-κB is the prototype of a family of dimeric transcription factors whose subunits contain conserved Rel regions [Verma et al., Genes Develop., 9:2723-2735 (1996); Baldwin, Ann. Rev. Immunol., 14:649-681 (1996)]. In its latent form, NF-κB is complexed with members of the IκB inhibitor family; upon inactivation of the IκB in response to certain stimuli, released NF-κB translocates to the nucleus where it binds to specific DNA sequences and activates gene transcription. As described above, the TNFR members identified to date either include or lack an intracellular death domain region. Some TNFR molecules lacking a death domain, such as TNFR2, CD40, HVEM, and GITR, are capable of modulating NF-κB activity. [see, e.g., Lotz et al., J. Leukocyte Biol., 60:1-7 (1996)].

For a review of the TNF family of cytokines and their receptors, see Ashkenazi and Dixit, Science, 281:1305-1308 (1998); Golstein, Curr. Biol., 7:750-753 (1997); Gruss and Dower, supra, and Nagata, Cell, 88:355-365 (1997).

### SUMMARY OF THE INVENTION

Applicants have surprisingly found that Apo-2 ligand or other Apo-2L receptor agonists and CPT-11 can act synergistically to induce apoptosis in mammalian cells, particularly in mammalian cancer cells.

The invention provides various methods for the use of Apo-2 ligand and CPT-11 to induce apoptosis in mammalian cells. For example, the invention provides a method for inducing apoptosis comprising exposing a mammalian cancer cell (preferably a colon or colorectal cancer cell), in cell culture or ex vivo, to Apo-2 ligand and CPT-11 in an amount effective to synergistically induce apoptosis. The invention further includes medicaments for treating a mammal suffering from cancer comprising an effective amount of Apo-2 ligand and CPT-11, as disclosed herein.

The agonistic anti-Apo-2 ligand receptor antibody may mimic the apoptotic activity of Apo-2 ligand. In a preferred embodiment, the agonist antibody will comprise a monoclonal antibody against the DR4 or DR5 receptor.

The invention also provides compositions which comprise Apo-2 ligand or Apo-2L receptor agonist antibody and/or CPT-11. Optionally, the compositions of the invention will include pharmaceutically acceptable carriers or diluents. Preferably, the compositions will include Apo-2 ligand or agonist antibody and/or CPT-11 in an amount which is effective to synergistically induce apoptosis in mammalian cells

The invention also provides articles of manufacture and kits which include Apo-2 ligand or Apo-2L receptor agonist antibody and/or CPT-11.

### BRIEF DESCRIPTION OF THE DRAWINGS

Figure 1 shows the effect of Apo-2L (open triangles), CPT-11 (open squares), Apo-2L plus CPT-11 (closed triangles), or vehicle alone (open circles) on growth of human colon carcinoma cells injected subcutaneously into athymic nude mice.
Figure 2 shows the effect of Apo-2L (60 mg/kg) (open squares), CPT-11 (80 mg/kg) (closed triangles), Apo-2L ("Apo2L.0") plus CPT-11 (closed squares), anti-DR4 mAb 4H6 (open triangles), anti-DR4 mAb plus CPT-11 (closed triangles) or vehicle alone (closed circles) on growth of human colon carcinoma cells injected subcutaneously into athymic nude mice.

### DETAILED DESCRIPTION OF THE INVENTION

### I. Definitions

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured using techniques known in the art, for instance, by cell viability assays, FACS analysis or DNA electrophoresis, and more specifically by binding of annexin V, fragmentation of DNA, cell shrinkage, dilation of endoplasmatic reticulum, cell fragmentation, and/or formation of membrane vesicles (called apoptotic bodies).

As used herein, the term "synergy" or "synergism" or "synergistically" refers to the interaction of two or more agents so that their combined effect is greater than the sum of their individual effects.

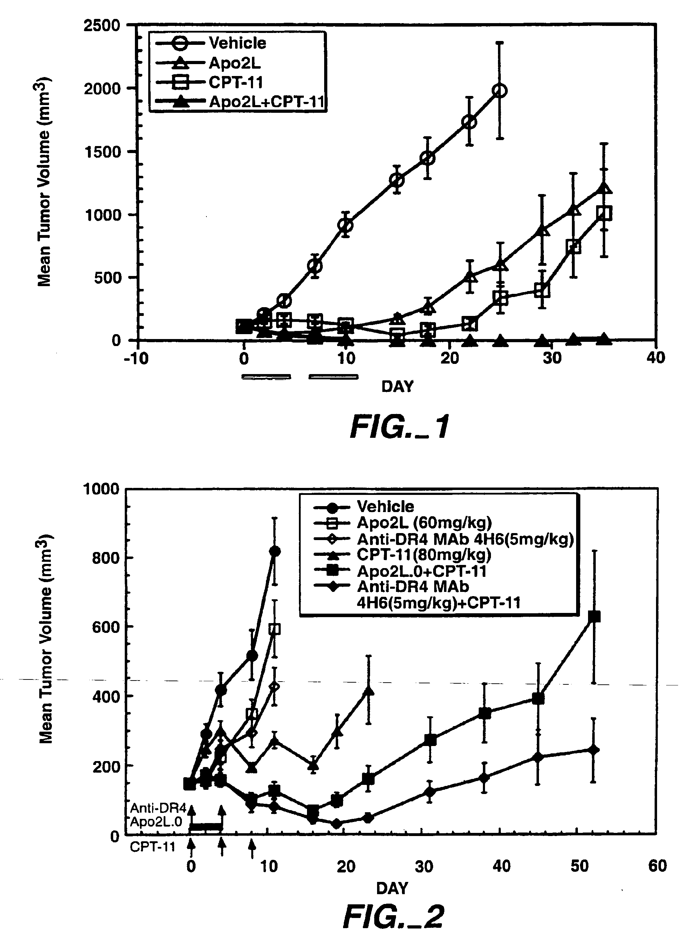

The terms "Apo-2 ligand", "Apo-2L", or "TRAIL" are used herein to refer to a polypeptide which includes amino acid residues 95-281, inclusive, 114-281, inclusive, residues 91-281, inclusive, residues 92-281, inclusive, residues 41-281, inclusive, residues 15-281, inclusive, or residues 1-281, inclusive, of the amino acid sequence shown in Figure 1A of Pitti et al., J. Biol. Chem., 271:12687-12690 (1996), as well as biologically active fragments, deletional, insertional, or substitutional variants of the above sequences. In one embodiment, the polypeptide sequence comprises residues 114-281. Optionally, the polypeptide sequence has at least residues 91-281 or residues 92-281. In another preferred embodiment, the biologically active fragments or variants have at least about 80% amino acid sequence identity, more preferably at least about 90% amino acid sequence identity, and even more preferably, at least about 95%, 96%, 97%, 98%, or 99% amino acid sequence identity with any one of the above sequences. The definition encompasses substitutional variants of the Apo-2 ligand comprising amino acids 91-281 of Figure 1A of Pitti et al., J. Biol. Chem., 271:12687-12690 (1996) in which at least one of the amino acids at positions 203, 218 or 269 (using the numbering of the sequence provided in Pitti et al., supra) are substituted by an alanine residue. The definition encompasses Apo-2 ligand isolated from an Apo-2 ligand source, such as from human tissue types, or from another source, or prepared by recombinant or synthetic methods. The term Apo-2 ligand also refers to the polypeptides described in WO 97/25428, supra, and WO97/01633, supra.

The term "CPT-11" refers to a chemotherapy or chemotherapeutic agent which is of the topoisomerase I inhibitor class. The term "CPT-11" as used herein includes the chemotherapeutic agents having the chemical name (4S)-4, 11-diethyl-4-hydroxy-9-[(4-piperidino-piperidino)carbonyloxyl]-IH-pyrano[3' , 4':6,7]indolizino [1, 2-b]quinoline-3, 14(4H, 12H)dione hydrochloride trihydrate, and the names irinotecan, camptothecin, topotecan, or Camptosar®, as well as water-soluble derivatives thereof or pharmaceutically acceptable salts of such agents. Irinotecan hydrochloride has the empirical formula C₃₃H₃₈N₄O₆*HCl*3H₂O and a molecular weight of approximately 677.19. Such chemical names and chemical formulae will be readily familiar to those skilled in the art. Camptosar® is commercially available from Pharmacia & Upjohn and approved for marketing in the United States by the FDA. The product insert for Camptosar® indicates the molecule can be used for treatment of human patients with metastatic colorectal carcinoma whose disease has recurred or progressed following 5-FU based therapy.

"Percent (%) amino acid sequence identity" with respect to the Apo-2L polypeptide sequences identified herein is defined as the percentage of amino acid residues in a candidate sequence that are identical with the amino acid residues in an Apo-2L sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. Optionally, % amino acid sequence identity values are obtained by using the sequence comparison computer program ALIGN-2. The ALIGN-2 sequence comparison computer program was authored by Genentech, Inc. and the source code has been filed with user documentation in the U.S. Copyright Office, Washington, D.C., 20559, where it is registered under U.S. Copyright Registration No. TXU510087. The ALIGN-2 program is publicly available through Genentech, Inc., South San Francisco, California. The ALIGN-2 program should be compiled for use on a UNIX operating system, preferably digital UNIX V4.0D. All sequence comparison parameters are set by the ALIGN-2 program and do not vary. However, % amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

The term "antibody" when used in reference to an "agonistic anti-Apo-2 ligand receptor antibody" is used in the broadest sense and specifically covers intact monoclonal antibodies, polyclonal antibodies, multispecific antibodies (e.g. bispecific antibodies) formed from at least two intact antibodies, and antibody fragments so long as they mimic the apoptotic activity of Apo-2 ligand.

"Apo-2 ligand receptor" includes the receptors referred to in the art as "DR4" and "DR5". Pan et al. have described the TNF receptor family member referred to as "DR4" [Pan et al., Science, 276:111-113 (1997); see also WO98/32856 published July 30, 1998]. The DR4 receptor was reported to contain a cytoplasmic death domain capable of engaging the cell suicide apparatus. Pan et al. disclose that DR4 is believed to be a receptor for the ligand known as Apo2L/TRAIL. Sheridan et al., Science, 277:818-821 (1997) and Pan et al., Science, 277:815-818 (1997) described another receptor for Apo2L/TRAIL [see also, WO98/51793 published November 19, 1998; WO98/41629 published September 24, 1998]. This receptor is referred to as DR5 (the receptor has also been alternatively referred to as Apo-2; TRAIL-R, TR6, Tango-63, hAP08, TRICK2 or KILLER; Screaton et al., Curr. Biol., 7:693-696 (1997); Walczak et al., EMBO J., 16:5386-5387 (1997); Wu et al., Nature Genetics, 17:141-143 (1997); WO98/3598 published August 20, 1998; EP870,827 published October 14, 1998; WO98/46643 published October 22, 1998; WO99/02653 published January 21, 1999; WO99/09165 published February 25, 1999; WO99/1179 published March 11, 1999]. Like DR4, DR5 is reported to contain a cytoplasmic death domain and be capable of signaling apoptosis. As described above, other receptors for Apo-2L include DcR1, DcR2, and OPG [see, Sheridan et al., supra; Marsters et al., supra; and Simonet et al., supra]. The term "Apo-2L receptor" when used herein encompasses native sequence receptor and receptor variants. These terms encompass Apo-2L receptor expressed in a variety of mammals, including humans. Apo-2L receptor may be endogenously expressed as occurs naturally in a variety of human tissue lineages, or may be expressed by recombinant or synthetic methods. A "native sequence Apo-2L receptor" comprises a polypeptide having the same amino acid sequence as an Apo-2L receptor derived from nature. Thus, a native sequence Apo-2L receptor can have the amino acid sequence of naturally-occurring Apo-2L receptor from any mammal. Such native sequence Apo-2L receptor can be isolated from nature or can be produced by recombinant or synthetic means. The term "native sequence Apo-2L receptor" specifically encompasses naturally-occurring truncated or secreted forms of the receptor (e.g., a soluble form containing, for instance, an extracellular domain sequence), naturally-occurring variant forms (e.g., alternatively spliced forms) and naturally-occurring allelic variants. Receptor variants may include fragments or deletion mutants of the native sequence Apo-2L receptor.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, e.g., U.S. Patent No. 4,816,567). The "monoclonal antibodies" may also be isolated from phage antibody libraries.using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)).

"Humanized" forms of non-human (*e*.*g*., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321:522-525 (1986); Reichmann et al., Nature, 332:323-329 (1988); and Presta, Curr. Op. Struct. Biol., 2:593-596 (1992). The humanized antibody includes a PRIMATIZED™ antibody wherein the antigen-binding region of the antibody is derived from an antibody produced by immunizing macaque monkeys with the antigen of interest.

Antibodies are typically proteins or polypeptides which exhibit binding specificity to a specific antigen. Native antibodies are usually heterotetrameric glycoproteins, composed of two identical light (L) chains and two identical heavy (H) chains. Typically, each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains [Chothia et al., J. Mol. Biol., 186:651-663 (1985); Novotny and Haber, Proc. Natl. Acad. Sci. USA, 82:4592-4596 (1985)]. The light chains of antibodies from any vertebrate species can be assigned to one of two clearly distinct types, called kappa and lambda, based on the amino acid sequences of their constant domains. Depending on-the amino acid-sequence of-the constant domain of their heavy chains, immunoglobulins can be assigned to different classes. There are five major classes of immunoglobulins: IgA, IgD, IgE, IgG and IgM, and several of these may be further divided into subclasses (isotypes), e.g., IgG-1, IgG-2, IgG-3, and IgG-4; IgA-1 and IgA-2. The heavy chain constant domains that correspond to the different classes of immunoglobulins are called alpha, delta, epsilon, gamma, and mu, respectively. "Antibody fragments" comprise a portion of an intact antibody, generally the antigen binding or variable region of the intact antibody. Examples of antibody fragments include Fab, Fab', F(ab')2, and Fv fragments, diabodies, single chain antibody molecules, and multispecific antibodies formed from antibody fragments.

The term "variable" is used herein to describe certain portions of the variable domains which differ in sequence among antibodies and are used in the binding and specificity of each particular antibody for its particular antigen. However, the variability is not usually evenly distributed through the variable domains of antibodies. It is typically concentrated in three segments called complementarity determining regions (CDRs) or hypervariable regions both in the light chain and the heavy chain variable domains. The more highly conserved portions of the variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies [see Kabat, E.A. et al., Sequences of Proteins of Immunological Interest, National Institutes of Health, Bethesda, MD (1987)]. The constant domains are not involved directly in binding an antibody to an antigen, but exhibit various effector functions, such as participation of the antibody in antibody-dependent cellular toxicity.

The monoclonal antibodies herein include chimeric, hybrid and recombinant antibodies produced by splicing a variable (including hypervariable) domain of an anti-Apo-2L receptor antibody with a constant domain (e.g. "humanized" antibodies), or a light chain with a heavy chain, or a chain from one species with a chain from another species, or fusions with heterologous proteins, regardless of species of origin or immunoglobulin class or subclass designation, as well as antibody fragments (e.g., Fab, F(ab')₂, and Fv), so long as they exhibit the desired biological activity or properties. See, e.g. U.S. Pat. No. 4,816,567 and Mage et al., in Monoclonal Antibody Production Techniques and Applications, pp.79-97 (Marcel Dekker, Inc.: New York, 1987).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et *al*. Nature Biotechnology, 14:309-314 (1996): Sheets et *al.* PNAS, *(USA)* 95:6157-6162 (1998)); Hoogenboom and Winter, J. Mol. Biol., 227:381 (1991); Marks et *al.,* J. Mol. Biol., 222:581 (1991)). Human antibodies can also be made by introducing human immunoglobulin loci into transgenic animals, e,g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology, 10: 779-783 (1992); Lonberg et al., Nature, 368: 856-859 (1994); Morrison, Nature, 368:812-13 (1994); Fishwild et al., Nature Biotechnology, 14: 845-51 (1996); Neuberger, Nature Biotechnology, 14: 826 (1996); Lonberg and Huszar, Intern. Rev. Immunol., 13:65-93 (1995). Alternatively, the human antibody may be prepared via immortalization of human B lymphocytes producing an antibody directed against a target antigen (such B lymphocytes may be recovered from an individual or may have been immunized *in vitro).* See, e.g., Cole et al., Monoclonal Antibodies and Cancer Therapy, Alan R. Liss, p. 77 (1985); Boerner et *al.,* J. Immunol., 147 (1):86-95 (1991); and US Pat No. 5,750,373.

The term "Fc region" is used to define the C-terminal region of an immunoglobulin heavy chain which may be generated by papain digestion of an intact antibody. The Fc region may be a native sequence Fc region or a variant Fc region. Although the boundaries of the Fc region of an immunoglobulin heavy chain might vary, the human IgG heavy chain Fc region is usually defined to stretch from an amino acid residue at about position Cys226, or from about position Pro230, to the carboxyl-terminus of the Fc region (using herein the numbering system according to Kabat et al., supra). The Fc region of an immunoglobulin generally comprises two constant domains, a CH2 domain and a CH3 domain, and optionally comprises a CH4 domain.

By "Fc region chain" herein is meant one of the two polypeptide chains of an Fc region.

The "CH2 domain" of a human IgG Fc region (also referred to as "Cγ2" domain) usually extends from an amino acid residue at about position 231 to an amino acid residue at about position 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, *Molec. Immunol*.22:161-206 (1985). The CH2 domain herein may be a native sequence CH2 domain or variant CH2 domain.

The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protroberance" in one chain thereof and a corresponding introduced "cavity" in the other chain thereof; see US Patent No. 5,821,333).

"Hinge region" is generally defined as stretching from about Glu216, or about Cys226, to about Pro230 of human IgG1 (Burton, *Molec. Immunol.*22:161-206 (1985)). Hinge regions of other IgG isotypes may be aligned with the IgG1 sequence by placing the first and last cysteine residues forming inter-heavy chain S-S bonds in the same positions. The hinge region herein may be a native sequence hinge region or a variant hinge region. The two polypeptide chains of a variant hinge region generally retain at least one cysteine residue per polypeptide chain, so that the two polypeptide chains of the variant hinge region can form a disulfide bond between the two chains. The preferred hinge region herein is a native sequence human hinge region, e.g. a native sequence human IgG1 hinge region.

A "functional Fc region" possesses at least one "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (e.g. B cell receptor; BCR), etc. Such effector functions generally require the Fc region to be combined with a binding domain (e.g. an antibody variable domain) and can be assessed using various assays known in the art for evaluating such antibody effector functions.

A "native sequence Fc region" comprises an amino acid sequence identical to the amino acid sequence of an Fc region found in nature. A "variant Fc region" comprises an amino acid sequence which differs from that of a native sequence Fc region by virtue of at least one amino acid modification. Preferably, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide. The variant Fc region herein will preferably possess at least about 80% sequence identity with a native sequence Fc region and/or with an Fc region of a parent polypeptide, and most preferably at least about 90% sequence identity therewith, more preferably at least about 95% sequence identity therewith.

The terms "Fc receptor" and "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (reviewed in Daëron, Annu. Rev. Immunol., 15:203-234 (1997)). FcRs are reviewed in Ravetch and Kinet, Annu. Rev. Immunol., 9:457-92 (1991); Capel et al., Immunomethods, 4:25-34 (1994); and de Haas et al., J. Lab. Clin. Med., 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et *al.,* J. Immunol., 117:587 (1976); and Kim et *al.,* J. Immunol., 24:249 (1994)).

An "affinity matured" antibody is one with one or more alterations in one or more CDRs thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et *al.* Bio/Technology, 10:779-783 (1992) describes affinity maturation by VH and VL domain shuffling. Random mutagenesis of CDR and/or framework residues is described by: Barbas et al. Proc Nat. Acad. Sci, *USA* 91:3809-3813 (1994); Schier et al. Gene, 169:147-155 (1995); Yelton et *al.* *J.* Immunol., 155:1994-2004 (1995); Jackson et *al.,* J. Immunol., 154(7):3310-9 (1995); and Hawkins et *al,* J. Mol. Biol., 226:889-896 (1992).

The terms "agonist" and "agonistic" when used herein refer to or describe a molecule which is capable of, directly or indirectly, substantially inducing, promoting or enhancing biological activity or activation of a receptor for Apo-2 ligand. Optionally, an "agonist Apo-2L receptor antibody" is an antibody which has activity that mimics or is comparable to Apo-2 ligand. Preferably, the agonist is a molecule which is capable of inducing apoptosis in a mammalian cell. Even more preferably, the agonist is an antibody directed to an Apo-2L receptor and said antibody has apoptotic activity which is equal to or greater than the Apo-2L polypeptide described in Example 1. Optionally, the agonist activity of such molecule can be determined by assaying the molecule, alone or in a cross-linked form using Fc immunoglobulin or complement (described below), in an assay described in Example 2 to examine apoptosis of 9D cells or other cells which express a receptor for Apo-2L such as DR4 or DR5.

"Isolated," when used to describe the various proteins disclosed herein, means protein that has been identified and separated and/or recovered from a component of its natural environment. Contaminant components of its natural environment are materials that would typically interfere with diagnostic or therapeutic uses for the protein, and may include enzymes, hormones, and other proteinaceous or non-proteinaceous solutes. In preferred embodiments, the protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated protein includes protein in situ within recombinant cells, since at least one component of the protein natural environment will not be present. Ordinarily, however, isolated protein will be prepared by at least one purification step.

"Biologically active" or "biological activity" for the purposes herein means (a) having the ability to induce or stimulate apoptosis in at least one type of mammalian cancer cell or virally-infected cell *in vivo* or *ex vivo;* (b) capable of raising an antibody, i.e., immunogenic; or (c) retaining the activity of a native or naturally-occurring Apo-2 ligand polypeptide.

A "growth inhibitory agent" when used herein refers to a compound or composition which inhibits growth of a cell in *vitro* and/or *in vivo.* Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), TAXOL®, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13.

The term "prodrug" as used in this application refers to a precursor or derivative form of a pharmaceutically active substance that is less cytotoxic to cancer cells compared to the parent drug and is capable of being enzymatically activated or converted into the more active parent - form. See, *e.g.*, Wilman, "Prodrugs in Cancer Chemotherapy" Biochemical. *Society Transactions,* 14, pp. 375-382, 615th Meeting Belfast (1986) and Stella et al., "Prodrugs: A Chemical Approach to Targeted Drug Delivery," *Directed Drug Delivery,* Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described below.

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of conditions like cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (Particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin γ₁^{I} and calicheamicin θ^{I} ₁, see, e.g., Agnew Chem Intl. Ed. Engl., 33:183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; podophyllinic acid; 2-ethylhydrazide; procarbazine; PSK®; razoxane; rhizoxin; sizofiran; spirogermanium; tenuazonic acid; triaziquone; 2, 2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL® , Bristol-Myers Squibb Oncology, Princeton, NJ) and doxetaxel (TAXOTERE® , Rhône-Poulenc Rorer, Antony, France); chlorambucil, gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and antiandrogens such as flutamide, nilutamide, bicalutamide, leuprolide, and goserelin; and pharmaceutically acceptable salts, acids or derivatives of any of the above.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and -beta; mullerian-inhibiting substance; mouse gonadotropin-associated peptide; inhibin; activin; vascular endothelial growth factor; integrin; thrombopoietin (TPO); nerve growth factors such as NGF-alpha; platelet-growth factor; transforming growth factors (TGFs) such as TGF-alpha and TGF-beta; insulin-like growth factor-I and -II; erythropoietin (EPO); osteoinductive factors; interferons such as interferon-alpha, -beta and -gamma colony stimulating factors (CSFs) such as macrophage-CSF (M-CSF); granulocyte-macrophage-CSF (GM-CSF); and granulocyte-CSF (G-CSF); interleukins (ILs) such as IL-1, IL-lalpha, IL-2, IL-3, IL-4, IL-5, IL-6, IL-7, IL-8, IL-9, IL-10, IL-11, IL-12; a tumor necrosis factor such as TNF-alpha or TNF-beta; and other polypeptide factors including LIF and kit ligand (KL). As used herein, the term cytokine includes proteins from natural sources or from recombinant cell culture and biologically active equivalents of the native sequence cytokines.

"Treatment" or "therapy" refer to both therapeutic treatment and prophylactic or preventative measures.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy *in vivo* can, for example, be measured by assessing tumor burden or volume, the time to disease progression (TTP) and/or determining the response rates (RR).

"Mammal" for purposes of treatment or therapy refers to any animal classified as a mammal, including humans, domestic and farm animals, and zoo, sports, or pet animals, such as dogs, horses, cats, cows, etc. Preferably, the mammal is human.

The terms "cancer", "cancerous", or "maligant" refer to or describe the physiological condition in mammals that is typically characterized by unregulated cell growth. Examples of cancer include but are not limited to, carcinoma, lymphoma, blastoma, sarcoma, and leukemia More particular examples of such cancers include colon cancer, colorectal cancer, rectal cancer, squamous cell cancer, small-cell lung cancer, non-small cell lung cancer, Hodgkin's and non-Hodgkin's lymphoma, testicular cancer, esophageal cancer, gastrointestinal cancer, renal cancer, pancreatic cancer, glioblastoma, cervical cancer, ovarian cancer, glioma, liver cancer, bladder cancer, hepatoma, breast cancer, endometrial carcinoma, salivary gland carcinoma, kidney cancer, liver cancer, prostate cancer, vulval cancer, thyroid cancer, hepatic carcinoma and various types of head and neck cancer.

### II. Methods and Materials

Generally, the methods of the invention for inducing apoptosis in mammalian cells comprise exposing the cells to an effective amount of Apo-2 ligand and CPT-11 or an effective amount of Apo-2L receptor agonist antibody and CPT-11. Preferably, the amount of Apo-2L (or agonist antibody) and CPT-11 employed will be amounts effective to synergistically induce apoptosis. This can be accomplished *in vivo* or *ex vivo* in accordance, for instance, with the methods described below and in the Examples. Exemplary conditions or disorders to be treated with the Apo-2 ligand or agonist antibody and CPT-11 include benign or malignant cancer.

### A. MATERIALS

The Apo-2L which can be employed in the methods includes the Apo-2L polypeptides described in Pitti et al., supra, WO 97/25428, supra, and WO97/01633, supra (the polypeptides referred to as TRAIL). It is contemplated that various forms of Apo-2L may be used, such as the full length polypeptide as well as soluble forms of Apo-2L which comprise an extracellular domain (ECD) sequence. Examples of such soluble ECD sequences include polypeptides comprising amino acids 114-281, 95-281, 91-281 or 92-281 of the Apo-2L sequence shown in Figure 1A of Pitti et al., J. Biol. Chem., 271:12687-12690 (1996). It is presently believed that the polypeptide comprising amino acids 92-281 is a naturally cleaved form of Apo-2L. Applicants have expressed human Apo-2L in CHO cells and found that the 92-281 polypeptide is the expressed form of Apo-2L. Modified forms of Apo-2L, such as the covalently modified forms described in WO 97/25428 are included. In particular, Apo-2L linked to a non-proteinaceous polymer such as polyethylene glycol is included for use in the present methods. The Apo-2L polypeptide can be made according to any of the methods described in WO 97/25428.

Variants of Apo-2 ligand having apoptotic activity which can be used in the methods include, for example, those identified by alanine scanning techniques. Particular substitutional variants comprise amino acids 91-281 of Figure 1A of Pitti et al., J. Biol. Chem., 271:12687-12690 (1996) in which at least one of the amino acids at positions 203, 218 or 269 are substituted by an alanine residue. Optionally, the Apo-2 ligand variants may include one or more of these three different site substitutions.

It is contemplated that a molecule which mimics the apoptotic activity of Apo-2L may alternatively be employed in the presently disclosed methods. Examples of such molecules include agonistic antibodies which can induce apoptosis in at least a comparable or like manner to Apo-2L. In particular, these agonist antibodies would comprise antibodies to one or more of the receptors for Apo-2L. Preferably, the agonist antibody is directed to an Apo-2L receptor which includes a cytoplasmic death domain such as DR4 or DR5. Even more preferably, the agonist antibody binds to such a receptor and binding can be determined, e.g., using FACS analysis or ELISA, such as described in Example 2. Agonist antibodies directed to the receptor called DR5 (or Apo-2) have been prepared using fusion techniques such as described below. One of the DR5 or Apo-2 receptor agonist antibodies is referred to as 3Fll.39.7 and has been deposited with ATCC as deposit no. HB-12456 on January 13, 1998. Other DR5 receptor antibodies include 3H3.14.5, deposited with ATCC as shown herein. Agonist activity of the Apo-2L receptor antibodies can be determined using various methods for assaying for apoptotic activity, and optionally, apoptotic activity of such antibody can be determined by assaying the antibody, alone or in a cross-linked form using Fc immunoglobulin or complement (described below), in the assay described in Example 2 to examine apoptosis of 9D cells or other cells expressing an Apo-2L receptor such as DR4 or DR5.

Additionally, agonist antibodies directed to another Apo-2L receptor called DR4 have also been prepared. One of the DR4 agonist antibodies is referred to as 4H6.17.8 and has been deposited with ATCC as deposit no. HB-12455 on January 13, 1998. Still further agonist DR4 antibodies include the antibodies 4E7.24.3, 1H5.25.9, 4G7.18.8, and 5G11.17.1 which have been deposited with ATCC, as shown below. Agonist activity of the Apo-2L receptor antibodies can be determined using various methods for assaying for apoptotic activity, and optionally, apoptotic activity of such antibody can be determined by assaying the antibody, alone or in a cross-linked form using Fc immunoglobulin or complement (described below), in the assay described in Example 2 to examine apoptosis of 9D cells or other cells expressing an Apo-2L receptor such as DR4 or DR5.

Agonist antibodies contemplated by the invention include antibodies which bind a single Apo-2L receptor or more than one Apo-2L receptor. An antibody which binds more than one Apo-2L receptor can be characterized as an antibody that "cross-reacts" with two or more different antigens and capable of binding to each of the different antigens, e.g. as determined by ELISA or FACS as in the examples below. Optionally, an antibody which "specifically cross-reacts" with two or more different antigens is one which binds to a first antigen and further binds to a second different antigen, wherein the binding ability of the antibody for the second antigen at an antibody concentration of about 10µg/mL is from about 50% to about 100% (preferably from about 75% to about 100%) of the binding ability of the first antigen as determined in a capture ELISA (such as in the examples below). For example, the antibody may bind specifically to DR5 (the "first antigen") and specifically cross-react with another Apo-2L receptor such as DR4 (the "second antigen"), wherein the extent of binding of about 10µg/mL of the antibody to DR4 is about 50% to about 100% of the binding ability of the antibody for DR5 in the capture ELISA herein. Various cross-reactive antibodies to Apo-2L receptors are described in further detail in International Patent application number PCT/US99/13197.

As described below, exemplary forms of such antibodies include polyclonal, monoclonal, humanized, bispecific, and heteroconjugate antibodies.

### 1. Polyclonal Antibodies

The antibodies of the invention may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include a DR4 or DR5 polypeptide (or a DR4 or DR5 ECD) or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation. The mammal can then be bled, and the serum assayed for antibody titer. If desired, the mammal can be boosted until the antibody titer increases or plateaus.

### 2. Monoclonal Antibodies

The antibodies of the invention may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include a DR4 or DR5 polypeptide or a fusion protein thereof, such as a DR4 or DR5 ECD-IgG fusion protein.

Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. An example of such a murine myeloma cell line is P3X63AgU.1 described in Example 2 below. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against the Apo-2L receptor. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium or RPMI-1640 medium. Alternatively, the hybridoma cells may be grown *in vivo* as ascites in a mammal.

The monoclonal antibodies secreted by the subclones may be isolated or purified from the culture medium or ascites fluid by_conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody. Optionally, chimeric antibodies can be constructed which include at least one variable or hypervariable domain of an anti-Apo-2L receptor antibody selected from the 4H6.17.8, 3F11.39.7, 4E7.24.3, 1H5.25.9, 4G7.18.8, 5G11.17.1, and 3H3.14.5 antibodies disclosed herein.

Optionally, the agonist antibodies of the present invention will bind to the same epitope(s) as any of the 4H6.17.8, 3F11.39.7, 4E7.24.3, 1H5.25.9, 4G7.18.8, 5G11.17.1, and 3H3.14.5 antibodies disclosed herein. This can be determined by conducting various assays, such as described herein. For instance, to determine whether a monoclonal antibody has the same specificity as the DR4 or DR5 antibodies specifically referred to herein, one can compare its activity in blocking assays or apoptosis induction assays.

The antibodies of the invention include "cross-linked" antibodies. The term "cross-linked" as used herein refers to binding of at least two IgG molecules together to form one (or single) molecule. The Apo-2L receptor antibodies may be cross-linked using various linker molecules, optionally the DR4 antibodies are cross-linked using an anti-IgG molecule, complement, chemical modification or molecular engineering. It is appreciated by those skilled in the art that complement has a relatively high affinity to antibody molecules once the antibodies bind to cell surface membrane. Accordingly, it is believed that complement may be used as a cross-linking molecule to link two or more antibodies bound to cell surface membrane. Among the various murine Ig isotypes, IgM; IgG2a and IgG2b are known-to fix complement.

The antibodies of the invention may optionally comprise dimeric antibodies, as well as multivalent forms of antibodies. Those skilled in the art may contruct such dimers or multivalent forms by techniques known in the art and using the anti-Apo-2L receptor antibodies herein.

The antibodies of the invention may also comprise monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published 12/22/94 and U.S. Patent No. 4,342,566. Papain digestion of antibodies typically produces two identical antigen binding fragments, called Fab fragments, each with a single antigen binding site, and a residual Fc fragment. Pepsin treatment yields an F(ab')₂ fragment that has two antigen combining sites and is still capable of cross-linking antigen.

The Fab fragments produced in the antibody digestion also contain the constant domains of the light chain and the first constant domain (CH₁) of the heavy chain. Fab' fragments differ from Fab fragments by the addition of a few residues at the carboxy terminus of the heavy chain CH₁ domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

Single chain Fv fragments may also be produced, such as described in Iliades et al., FEBS Letters, 409:437-441 (1997). Coupling of such single chain fragments using various linkers is described in Kortt et al., Protein Engineering, 10:423-433 (1997).

In addition to the antibodies described above, it is contemplated that chimeric or hybrid antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate.

The Apo-2L receptor antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human.variable domain has been substituted by the corresponding sequence from a non-human species. In practice, humanized antibodies are typically human antibodies in which some CDR residues and possibly some FR residues are substituted by residues from analogous sites in rodent antibodies. Sources of such import residues or import variable domains (or CDRs) include the deposited anti-Apo-2L receptor antibodies 4H6.17.8, 3F11.39.7, 4E7.24.3, 1H5.25.9, 4G7.18.8, 5G11.17.1, and 3H3.14.5 disclosed herein.

The choice of human variable domains, both light and heavy, to be used in making the humanized antibodies is very important in order to reduce antigenicity. According to the "best-fit" method, the sequence of the variable domain of a rodent antibody is screened against the entire library of known human variable domain sequences. The human sequence which is closest to that of the rodent is then accepted as the human framework (FR) for the humanized antibody [Sims et al., J. Immunol*.*, 151:2296-2308 (1993); Chothia and Lesk, J. Mol. Biol., 196:901-917 (1987)]. Another method uses a particular framework derived from the consensus sequence of all human antibodies of a particular subgroup of light or heavy chains. The same framework may be used for several different humanized antibodies [Carter et al., Proc. Natl. Acad. Sci. USA, 89:4285-4289 (1992); Presta et al., J. Immunol., 151:2623-2632 (1993)].

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three dimensional models of the parental and humanized sequences. Three dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding [see, WO 94/04679 published 3 March 1994].

Human monoclonal antibodies may be made via an adaptation of the hybridoma method first described by Kohler and Milstein by using human B lymphocytes as the fusion partner. Human B lymphocytes producing an antibody of interest may, for example, be isolated from a human individual, after obtaining informed consent. For instance, the individual may be producing antibodies against an autoantigen as occurs with certain disorders such as systemic lupus erythematosus (Shoenfeld et *al.* J. Clin. Invest., 70:205 (1982)), immune-mediated thrombocytopenic purpura (ITP) (Nugent et al. Blood, 70(1):16-22 (1987)), or cancer. Alternatively, or additionally, lymphocytes may be immunized *in vitro*. For instance, one may expose isolated human periperal blood lymphocytes *in vitro* to a lysomotrophic agent (e.g. L-leucine-0-methyl ester, L-glutamic acid dimethly ester or L-leucyl-L-leucine-O-methyl ester) (US Patent No. 5,567,610, Borrebaeck et al.); and/or T-cell depleted human peripheral blood lymphocytes may be treated *in vitro* with adjuvants such as 8-mercaptoguanosine and cytokines (US Patent No. 5,229,275, Goroff *et al*.).

The B lymphocytes recovered from the subject or immunized *in vitro,* are then generally immortalized in order to generate a human monoclonal antibody. Techniques for immortalizing the B lymphocyte include, but are not limited to: (a) fusion of the human B lymphocyte with human, murine myelomas or mouse-human heteromyeloma cells; (b) viral transformation (e.g. with an Epstein-Barr virus; see Nugent et al., *supra,* for example); (c) fusion with a lymphoblastoid cell line; or (d) fusion with lymphoma cells.

Lymphocytes may be fused with myeloma cells using a suitable fusing agent, such as polyethylene glycol, to form a hybridoma cell (Goding, Monoclonal Antibodies: Principles and Practice, pp.59-103 (Academic Press, 1986)). The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells. Suitable human myeloma and mouse-human heteromyeloma cell lines have been described (Kozbor, *J. Immunol.,* 133:3001 (1984); Brodeur et al., Monoclonal Antibody Production Techniques and Applications, pp. 51-63 (Marcel Dekker, Inc., New York, 1987)). Culture medium in which hybridoma cells are growing is assayed for production of monoclonal antibodies directed against the antigen. Preferably, the binding specificity of monoclonal antibodies produced by hybridoma cells is determined by immunoprecipitation or by an in *vitro* binding assay, such as radioimmunoassay (RIA) or enzyme-linked immunoabsorbent assay (ELISA).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. The monoclonal antibodies secreted by the subclones are suitably separated from the culture medium, ascites fluid, or serum by conventional immunoglobulin purification procedures such as, for example, protein A chromatography, gel electrophoresis, dialysis, or affinity chromatography.

Human antibodies may also be generated using a non-human host, such as a mouse, which is capable of producing human antibodies. As noted above, transgenic mice are now available that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g.*, Jakobovits *et al., Proc. Natl. Acad. Sci. USA,* 90:2551 (1993); Jakobovits *et al., Nature,* 362:255-258 (1993); Bruggermann *et al., Year in Immuno.,* 7:33 (1993); US Patent No. 5,591,669; US Patent No. 5,589,369; and US Patent No. 5,545,807. Human antibodies may also be prepared using SCID-hu mice (Duchosal *et al. Nature* 355:258-262 (1992)).

In another embodiment, the human antibody may be selected from a human antibody phage display library. The preparation of libraries of antibodies or fragments thereof is well known in the art and any of the known methods may be used to construct a family of transformation vectors which may be introduced into host cells. Libraries of antibody light and heavy chains in phage (Huse *et al., Science,* 246:1275 (1989)) or of fusion proteins in phage or phagemid can be prepared according to known procedures. See, for example, Vaughan *et al.,* Nature *Biotechnology* 14:309-314 (1996); Barbas *et al., Proc. Natl. Acad. Sci., USA,* 88:7978-7982 (1991); Marks *et al., J*. *Mol. Biol.,* 222:581-597 (1991); Hoogenboom and Winter, J. *Mol. Biol.,* 227:381-388 (1992); Barbas *et al., Proc. Natl. Acad. Sci., USA,* 89:4457-4461 (1992); Griffiths *et al*., *EMBO Journal,* 13:3245-3260 (1994); de Kruif *et al., J. Mol. Biol.,* 248:97-105 (1995); WO 98/05344; WO 98/15833; WO 97/47314; WO 97/44491; WO 97/35196; WO 95/34648; US Patent No. 5,712.089; US Patent No. 5,702,892; US Patent No. 5,427,908; US Patent No. 5,403,484; US Patent No. 5,432,018; US Patent No. 5,270,170; WO 92/06176; WO 99/06587; US Patent No. 5,514,548; WO97/08320; and US Patent No. 5,702,892. The antigen of interest is panned against the phage library using procedures known in the field for selecting phage-antibodies which bind to the target antigen.

The Apo-2L receptor antibodies, as described herein, will optionally possess one or more desired biological activities or properties. Such antibodies may include but are not limited to chimeric, humanized, human, and affinity matured antibodies. As described above, the antibodies may be constructed or engineered using various techniques to achieve these desired activities or properties. In one embodiment, the Apo-2L receptor antibody will have a DR4 or DR5 receptor binding affinity of at least 10⁵ M⁻¹, preferably at least in the range of 10⁶ M⁻¹ to 10⁷ M⁻¹ , more preferably, at least in the range of 10⁸ M to 10¹² M⁻¹ and even more preferably, at least in the range of 10⁹ M⁻¹ to 10¹² M⁻¹. The binding affinity of the antibody can be determined without undue experimentation by testing the antibody in accordance with techniques known in the art, including Scatchard analysis (see Munson et al., supra). For example, a DR4 antibody can be assayed for binding affinity to the DR4-IgG receptor construct, as described in Example 2.

In another embodiment, the Apo-2L receptor antibody of the invention may bind the same epitope on DR4 or DR5 to which Apo-2L binds, or bind an epitope on DR4 or DR5 which coincides or overlaps with the epitope on DR4 or DR5, respectively, to which Apo-2L binds. The antibody may also interact in such a way to create a steric conformation which prevents Apo-2 ligand binding to DR4 or DR5. The epitope binding property of the antibody of the present invention may be determined using techniques known in the art. For instance, the antibody may be tested in an in *vitro* assay, such as a competitive inhibition assay, to determine the ability of the antibody to block or inhibit binding of Apo-2L to DR4 or DR5. Optionally, the antibody may be tested in a competitive inhibition assay to determine the ability of, e.g., a DR4 antibody to inhibit binding of an Apo-2L polypeptide (such as described in Example 1) to a DR4-IgG construct (such as described in Example 2) or to a cell expressing DR4. Optionally, the antibody will be capable of blocking or inhibiting binding of Apo-2L to the receptor by at least 50%, preferably by at least 75% and even more preferably by at least 90%, which may be determined, by way of example, in an *in vitro* competitive inhibition assay using a soluble form of Apo-2 ligand (TRAIL) and a DR4 ECD-IgG (such as described in Example 2).

In a preferred embodiment, the antibody will comprise an agonist antibody having activity which mimics or is comparable to Apo-2 ligand (TRAIL). Preferably, such an agonistic DR4 or DR5 antibody will induce apoptosis in at least one type of cancer or tumor cell line or primary tumor. The apoptotic activity of an agonistic DR4 or DR5 antibody may be determined using known *in vitro* or in *vivo* assays. Examples of such *in vitro* and *in vivo* assays are described in detail in the Examples section below. *In vitro,* apoptotic activity can be determined using known techniques such as Annexin V binding. *In vivo,* apoptotic activity may be determined, e.g., by measuring reduction in tumor burden or volume.

### 3. Bispecific Antibodies

Bispecific antibodies are monoclonal, preferably human or humanized, antibodies that have binding specificities for at least two different antigens. In the present case, one of the binding specificities is for an Apo-2L receptor, the other one is for any other antigen, and preferably for a cell-surface protein or receptor or receptor subunit.

Methods for making bispecific antibodies are known in the-art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

### 4. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4, 676, 980.

### 5. Triabodies

Triabodies are also within the scope of the invention. Such antibodies are described for instance in Iliades et al., supra and Kortt et al., supra.

### 6. Other Modifications

Other modifications of the Apo-2L receptor antibodies are contemplated herein. The antibodies of the present invention may be modified by conjugating the antibody to a cytotoxic agent (like a toxin molecule) or a prodrug-activating enzyme which converts a prodrug (e.g. a peptidyl chemotherapeutic agent, see WO81/01145) to an active anti-cancer drug. See, for example, WO 88/07378 and U.S. Patent No. 4,975,278. This technology is also referred to as "Antibody Dependent Enzyme Mediated Prodrug Therapy" (ADEPT).

The enzyme component of the immunoconjugate useful for ADEPT includes any enzyme capable of acting on a prodrug in such a way so as to covert it into its more active, cytotoxic form. Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; caspases such as caspase-3; D-alanylcarboxypeptidases, useful for converting prodrugs that contain D-amino acid substituents; carbohydrate-cleaving enzymes such as beta-galactosidase and neuraminidase useful for converting glycosylated prodrugs into free drugs; beta-lactamase useful for converting drugs derivatized with beta-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, e.g., Massey, *Nature* 328: 457-458 (1987)). Antibody-abzyme conjugates can be prepared as described herein for delivery of the abzyme to a tumor cell population.

The enzymes can be covalently bound to the antibodies by techniques well known in the art such as the use of heterobifunctional crosslinking reagents. Alternatively, fusion proteins comprising at least the antigen binding region of an antibody of the invention linked to at least a functionally active portion of an enzyme of the invention can be constructed using recombinant DNA techniques well known in the art (see, e.g., Neuberger *et al., Nature,* 312: 604-608 (1984).

Further antibody modifications are contemplated. For example, the antibody may be linked to one of a variety of nonproteinaceous polymers, e.g., polyethylene glycol, polypropylene glycol, polyoxyalkylenes, or copolymers of polyethylene glycol and polypropylene glycol. The antibody also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in *Remington's Pharmaceutical Sciences,* 16th edition, Oslo, A., Ed., (1980). To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (e.g., IgG₁, IgG₂, IgG₃, or IgG₄) that is responsible for increasing the *in vivo* serum half-life of the IgG molecule.

### 7. Recombinant Methods

The invention also provides isolated nucleic acids encoding the antibodies as disclosed herein, vectors and host cells comprising the nucleic acid, and recombinant techniques for the production of the antibody.

For recombinant production of the antibody, the nucleic acid encoding it is isolated and inserted into a replicable vector for further cloning (amplification of the DNA) or for expression. DNA encoding the antibody is readily isolated and sequenced using conventional procedures (e.g., by using oligonucleotide probes that are capable of binding specifically to genes encoding the antibody). Many vectors are available. The vector components generally include, but are not limited to, one or more of the following: a signal sequence, an origin of replication, one or more marker genes, an enhancer element, a promoter, and a transcription termination sequence.

The methods herein include methods for the production of chimeric or recombinant anti-Apo-2L receptor antibodies which comprise the steps of providing a vector comprising a DNA sequence encoding an anti-Apo-2L receptor antibody light chain or heavy chain (or both a light chain and a heavy chain), transfecting or transforming a host cell with the vector, and culturing the host cell(s) under conditions sufficient to produce the recombinant anti-Apo-2L receptor antibody product.

### (i) Signal sequence component

The anti-Apo-2L receptor antibody of this invention may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (i.e., cleaved by a signal peptidase) by the host cell. For prokaryotic host cells that do not recognize and process the native antibody signal sequence, the signal sequence is substituted by a prokaryotic signal sequence selected, for example, from the group of the alkaline phosphatase, penicillinase, 1pp, or heat-stable enterotoxin II leaders. For yeast secretion the native signal sequence may be substituted by, e.g., the yeast invertase leader, α factor leader (including *Saccharomyces* and *Kluyveromyces* α-factor leaders), or acid phosphatase leader, the C. *albicans* glucoamylase leader, or the signal described in WO 90/13646. In mammalian cell expression, mammalian signal sequences as well as viral secretory leaders, for example, the herpes simplex gD signal, are available.

The DNA for such precursor region is ligated in reading frame to DNA encoding the antibody.

### (ii) Origin of replication component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

### (iii) Selection gene component

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II, preferably primate metallothionein genes, adenosine deaminase, ornithine decarboxylase, etc.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity.

Alternatively, host cells (particularly wild-type hosts that contain endogenous DHFR) transformed or co-transformed with DNA sequences encoding the anti-Apo-2L receptor antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Patent No. 4,965,199.

A suitable selection gene for use in yeast is the trp1 gene present in the yeast plasmid YRp7 (Stinchcomb *et al., Nature,* 282:39 (1979)). The *trp*1 gene provides a selection marker for a mutant strain of yeast lacking the ability to grow in tryptophan, for example, ATCC No. 44076 or PEP4-1. Jones, *Genetics,* 85:12 (1977). The presence of the *trp*1 lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the *Leu2* gene.

In addition, vectors derived from the 1.6 µm circular plasmid pKD1 can be used for transformation of *Kluyveromyces* yeasts. Alternatively, an expression system for large-scale production of recombinant calf chymosin was reported for *K. lactis.* Van den Berg, *Bio*/*Technology,* 8:135 (1990). Stable multi-copy expression vectors for secretion of mature recombinant human serum albumin by industrial strains of *Kluyveromyces* have also been disclosed. Fleer *et al., Bio*/*Technology,* 9:968-975 (1991).

### (iv) Promoter component

Expression and cloning vectors usually contain a promoter that is recognized by the host organism and is operably linked to the antibody nucleic acid. Promoters suitable for use with prokaryotic hosts include the *phoA* promoter, β-lactamase and lactose promoter systems, alkaline phosphatase, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding the anti-Apo-2L receptor antibody.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Anti-Apo-2L receptor antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes et *al., Nature* 297:598-601 (1982) on expression of human β-interferon cDNA in mouse cells under the control of a thymidine kinase promoter from herpes simplex virus. Alternatively, the rous sarcoma virus long terminal repeat can be used as the promoter.

### (v) Enhancer element component

Transcription of a DNA encoding the anti-Apo-2L receptor antibody of this invention by higher eukaryotes is often increased by inserting an enhancer sequence into the vector. Many enhancer sequences are now known from mammalian genes (globin, elastase, albumin, a-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the antibody-encoding sequence, but is preferably located at a site 5' from the promoter.

### (vi) Transcription termination component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding the multivalent antibody. One useful transcription termination component is the bovine growth hormone polyadenylation region. See WO94/11026 and the expression vector disclosed therein.

### (vii) Selection and transformation of host cells

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia, e.g., E*. *coli*, *Enterobacter, Erwinia, Klebsiella, Proteus, Salmonella, e.g., Salmonella typhimurium, Serratia, e.g., Serratia marcescans,* and *Shigella,* as well as Bacilli such as *B. subtilis* and *B. licheniformis (e.g., B. licheniformis* 41P disclosed in DD 266,710 published 12 April 1989), *Pseudomonas* such as *P. aeruginosa,* and *Streptomyces.* One preferred *E. coli* cloning host is *E. coli* 294 (ATCC 31,446), although other strains such as *E. coli* B, *E. coli* X1776 (ATCC 31,537), and *E. coli* W3110 (ATCC 27,325) are suitable. These examples are illustrative rather than limiting.

In addition to prokaryotes, eukaryotic microbes such as filamentous fungi or yeast are suitable cloning or expression hosts for Apo-2L receptor antibody-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe; Kluyveromyces* hosts such as, *e.g., K. lactis, K. fragilis* (ATCC 12,424), *K*. *bulgaricus* (ATCC 16,045), *K. wickeramii* (ATCC 24,178), *K*. *waltii* (ATCC 56,500), *K. drosophilarum* (ATCC 36,906), *K* . *thermotolerans,* and *K. marxianus; yarrowia* (EP 402,226); *Pichia pastoris* (EP 183,070); *Candida; Trichoderma reesia* (EP 244,234); *Neurospora crassa; Schwanniomyces* such as *Schwanniomyces occidentalis;* and filamentous fungi such as, e.g., *Neurospora, Penicillium, Tolypocladium,* and *Aspergillus* hosts such as *A*. *nidulans* and *A*. *niger.*

Suitable host cells for the expression of glycosylated antibody are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila* melanogaster (fruitfly), and *Bombyx mori* have been identified. A variety of viral strains for transfection are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham *et al., J. Gen Virol.* 36:59 (1977)); baby hamster kidney cells (BHK, ATCC CCL 10); Chinese hamster ovary cells/-DHFR (CHO, Urlaub *et al., Proc. Natl. Acad. Sci. USA* 77:4216 (1980)); mouse sertoli cells (TM4, Mather, *Biol. Reprod.* 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather *et al., Annals N.Y. Acad.* Sci. 383:44-68 (1982)); MRC 5 cells; FS4 cells; a human hepatoma line (Hep G2); and myeloma or lymphoma cells (e.g. Y0, J558L, P3 and NS0 cells) (see US Patent No. 5,807,715).

Host cells are transformed with the above-described expression or cloning vectors for antibody production and cultured in conventional nutrient media modified as appropriate for inducing promoters, selecting transformants, or amplifying the genes encoding the desired sequences.

### (viii) Culturing the host cells

The host cells used to produce the antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et *al., Meth. Enz.* 58:44 (1979), Barnes et *al.,* Anal. Biochem.102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Purification

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., *Bio*/*Technology* 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc region that is present in the antibody. Protein A can be used to purify antibodies that are based on human γ1, γ2, or γ4 heavy chains (Lindmark *et al., J. Immunol. Meth.* 62:1-13 (1983)). Protein G is recommended for all mouse isotypes and for human γ3 (Guss *et al., EMBO J.* 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C_{H}3 domain, the Bakerbond ABX™ resin (J. T. Baker, Phillipsburg, NJ) is useful for purification. Other techniques for protein purification such as fractionation on an ion-exchange column, ethanol precipitation. Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

### B. FORMULATIONS

The Apo-2 ligand or Apo-2L receptor agonist antibody and CPT-11 are preferably administered in a carrier. The molecules can be administered in a single carrier, or alternatively, can be included in separate carriers. Suitable carriers and their formulations are described in Remington's Pharmaceutical Sciences, 16th ed., 1980, Mack Publishing Co., edited by Oslo et al. Typically, an appropriate amount of a pharmaceutically-acceptable salt is used in the carrier to render the formulation isotonic. Examples of the carrier include saline, Ringer's solution and dextrose solution. The pH of the solution is preferably from about 5 to about 8, and more preferably from about 7.4 to about 7.8. It will be apparent to those persons skilled in the art that certain carriers may be more preferable depending upon, for instance, the route of administration and concentration of agent being administered. The carrier may be in the form of a lyophilized formulation or aqueous solution.

Acceptable carriers, excipients, or stabilizers are preferably nontoxic to cells and/or recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; and/or non-ionic surfactants such as TWEEN™, PLURONICS™ or polyethylene glycol (PEG).

The formulation may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The Apo-2L or agonist antibody and CPT-11 may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Oslo, A. Ed. (1980).

The formulations to be used for in *vivo* administration should be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly (2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

### C. MODES OF ADMINISTRATION

The Apo-2L or Apo-2L receptor agonist antibody and CPT-11 can be administered in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Optionally, administration may be performed through mini-pump infusion using various commercially available devices.

Effective dosages and schedules for administering Apo-2 ligand or agonist antibody and CPT-11 may be determined empirically, and making such determinations is within the skill in the art. It is presently believed that an effective dosage or amount of Apo-2 ligand used alone may range from about 1 µg/kg to about 100 mg/kg of body weight or more per day. An effective dosage or amount of CPT-11 used alone may range from about 1 mg/m² to about 150 mg/m². Interspecies scaling of dosages can be performed in a manner known in the art, e.g., as disclosed in Mordenti et al., Pharmaceut. Res., 8:1351 (1991). Those skilled in the art will understand that the dosage of Apo-2 ligand or agonist antibody and CPT-11 that must be administered will vary depending on, for example, the mammal which will receive the Apo-2 ligand or agonist antibody and CPT-11, the route of administration, and other drugs or therapies being administered to the mammal.

Depending on the type of cells and/or severity of the disease, about 1 µg/kg to 15 mg/kg (e.g. 0.1-20 mg/kg) of agonist antibody is an initial candidate dosage for administration, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful.

It is contemplated that yet additional therapies may be employed in the methods. The one or more other therapies may include but are not limited to, other chemotherapies (or chemotherapeutic agents) and/or radiation therapy, immunoadjuvants, growth inhibitory agents, cytokines, and other non-Her-2 antibody-based therapies. Examples include interleukins (*e.g.*, IL-1, IL-2, IL-3, IL-6), leukemia inhibitory factor, interferons, TGF-beta, erythropoietin, thrombopoietin, and anti-VEGF antibody. Other agents known to induce apoptosis in mammalian cells may also be employed, and such agents include TNF-α, TNF-β (lymphotoxin-α), CD30 ligand, 4-1BB ligand, and Apo-1 ligand.

Additional chemotherapies contemplated by the invention include chemical substances or drugs which are known in the art and are commercially available, such as Adriamycin, Doxorubicin, 5-Fluorouracil, Cytosine arabinoside ("Ara-C"), Cyclophosphamide, Leucovorin, Thiotepa, Busulfan, Cytoxin, Taxol, Toxotere, Methotrexate, Cisplatin, Melphalan, Vinblastine, Bleomycin, Etoposide, Ifosfamide, Mitomycin C, Mitoxantrone, Vincreistine, Vinorelbine, Carboplatin, Teniposide, Daunomycin, Carminomycin, Aminopterin, Dactinomycin, Mitomycins, Esperamicins (see U.S. Pat. No. 4,675,187), Melphalan and other related nitrogen mustards. Also included are agents that act to regulate or inhibit hormone action on tumors such as tamoxifen and onapristone.

Preparation and dosing schedules for such chemotherapy may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration with the Apo-2L or agonist antibody and/or CPT-11 or may be given simultaneously therewith.

The chemotherapy is preferably administered in a carrier, such as those described above. The mode of administration of the chemotherapy may be the same as employed for the Apo-2 ligand or agonist antibody or CPT-11 or it may be administered via a different mode.

Radiation therapy can be administered according to protocols commonly employed in the art and known to the skilled artisan. Such therapy may include cesium, iridium, iodine, or cobalt radiation. The radiation therapy may be whole body irradiation, or may be directed locally to a specific site or tissue in or on the body. Typically, radiation therapy is administered in pulses over a period of time from about 1 to about 2 weeks. The radiation therapy may, however, be administered over longer periods of time. Optionally, the radiation therapy may be administered as a single dose or as multiple, sequential doses.

The Apo-2 ligand or agonist antibody and CPT-11 (and one or more other therapies) may be administered concurrently or sequentially. Following administration of Apo-2 ligand or agonist antibody and CPT-11, treated cells *in vitro* can be analyzed. Where there has been in *vivo* treatment, a treated mammal can be monitored in various ways well known to the skilled practitioner. For instance, tumor mass may be observed physically, by biopsy or by standard x-ray imaging techniques.

### III. Articles of Manufacture

In another embodiment of the invention, an article of manufacture containing materials useful for the treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). The active agents in the composition are the Apo-2 ligand or agonist antibody and CPT-11. The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

The following example is offered by way of illustration and not by way of limitation. The disclosures of all citations in the specification are expressly incorporated herein by reference.

### EXAMPLE 1

This example illustrates the synergistic inhibition of tumor growth by Apo-2 ligand and CPT-11 *in vivo.*

The colon carcinoma cell line COLO205 (available from NCI) were grown and maintained according to the supplier's methods. Briefly, COLO205 cells were cultured in high glucose DMEM/F12 (50:50) media containing 10% fetal bovine serum and 2.0mM L-Glutamine. Apo-2 ligand comprising amino acids 114-281 was prepared in *E*. *coli .* The extracellular portion of human Apo-2L (amino acids 114-281; see Pitti et al., supra) was subcloned into the pS1346 expression plasmid (Scholtissek et al., Gene, 62:55-64 (1988)) with an added initiator methionine codon, and expressed under control of the trp promoter in *E. coli* strain W3110, in 10L or 100 L fermentors. Cell-paste containing recombinant human soluble Apo-2L was extracted with a buffer containing 0.1M Tris/0.2M NaCl/50 mM EDTA, pH 8.0. The extract was precipitated by 40% ammonium sulfate. Purification to >98 % homogeneity was achieved by two consecutive chromatographic separation steps on hydroxyapatite and Ni-NTA agarose columns. (Although it lacks a polyhistidine tag, the recombinant soluble 114-281 Apo-2L fragment is believed to bind to the Ni-NTA column through endogenous histidine residues). Purity was determined by sodium-dodecyl sulfate (SDS)-polyacrylamide gel electrophoresis and silver-nitrate or coomassie-blue staining, by amino acid sequence analysis, and by size-exclusion on high performance liquid chromatography (HPLC). CPT-11 (Camptosar®) was obtained from Pharmacia & Upjohn.

Athymic nude mice (Jackson Laboratories) were injected subcutaneously with 5 million COL0205 colon carcinoma cells and the tumors allowed to grow to about 120 mm³. Tumor-bearing mice were randomized into 4 groups at 9 mice per group and treated with either vehicle (20mM Tris, 8% Trehalose, 0.01 Tween-20, pH 7.5), Apo-2L (30 mg/kg/day on days 0-4 and 7-11), or CPT-11 (80 mg/kg/day on days 0, 4, and 8), or a combination of Apo-2L (30 mg/kg/day on days 0-4 and 7-11) plus CPT-11 (80 mg/kg/day on days 0, 4, and 8). Tumor volumes were determined at the indicated days over 34 days.

As shown in Figure 1, Apo-2L (open triangles) or CPT-11 (open squares) each suppressed tumor growth during the treatment period, although tumor growth resumed several days later in all 9 animals of each group. In contrast, the combination of Apo-2L with CPT-11(closed triangles) caused substantial tumor shrinkage, resulting in complete tumor elimination in 8 out of 9 animals in the combination treatment group.

The results of this experiment indicate that combinations of Apo-2 ligand and CPT-11 treatment synergistically inhibit growth of cancer cells *in vivo.*

### EXAMPLE 2

This example illustrates the synergistic inhibition of tumor growth by the DR4 receptor agonist antibody, 4H6.17.8 ("4H6"), and CPT-11 *in vivo.*

The agonist antibody was prepared as follows. A soluble DR4 ECD immunoadhesin construct was prepared. A mature DR4 ECD sequence (amino acids 1-218 shown in Pan et al., supra) was cloned into a pCMV-1 Flag vector (Kodak) downstream of the Flag signal sequence and fused to the CH1, hinge and Fc region of human immunoglobulin G₁ heavy chain as described previously [Aruffo et al., Cell, 61:1303-1313 (1990)]. The immunoadhesin was expressed by transient transfection into human 293 cells and purified from cell supernatants by protein A affinity chromatography, as described by Ashkenazi et al., Proc. Natl. Acad. Sci., 88:10535-10539 (1991).

Balb/c mice (obtained from Charles River Laboratories) were immunized by injecting 0.5 µg/50 µl of a DR4 ECD immunoadhesin protein (as described above)(diluted in MPL-TDM adjuvant purchased from Ribi Immunochemical Research Inc., Hamilton, MT) 11 times into each hind foot pad at 3-4 day intervals.

Three days after the final boost, popliteal lymph nodes were removed from the mice and a single cell suspension was prepared in DMEM media (obtained from Biowhitakker Corp.) supplemented with 1% penicillin-streptomycin. The lymph node cells were then fused with murine myeloma cells P3X63AgU. 1 (ATCC CRL 1597) using 35% polyethylene glycol and cultured in 96-well culture plates. Hybridomas resulting from the fusion were selected in HAT medium. Ten days after the fusion, hybridoma culture supernatants were screened in an ELISA to test for the presence of monoclonal antibodies binding to the DR4 ECD immunoadhesin protein (described above).

In the ELISA, 96-well microtiter plates (Maxisorp; Nunc, Kamstrup, Denmark) were coated by adding 50 µl of 2 µg/ml goat anti-human IgG Fc (purchased from Cappel Laboratories) in PBS to each well and incubating at 4°C overnight. The plates were then washed three times with wash buffer (PBS containing 0.05% Tween 20). The wells in the microtiter plates were then blocked with 200 µl of 2.0% bovine serum albumin in PBS and incubated at room temperature for 1 hour. The plates were then washed again three times with wash buffer.

After the washing step, 50 µl of 0.4 µg/ml DR4 ECD immunoadhesin protein in assay buffer was added to each well. The plates were incubated for 1 hour at room temperature on a shaker apparatus, followed by washing three times with wash buffer.

Following the wash steps, 100 µl of the hybridoma supernatants or Protein G-sepharose column purified antibody (10 µg/ml) was added to designated wells. 100 µl of P3X63AgU.1 myeloma cell conditioned medium was added to other designated wells as controls. The plates were incubated at room temperature for 1 hour on a shaker apparatus and then washed three times with wash buffer.

Next, 50 µl HRP-conjugated goat anti-mouse IgG Fc (purchased from Cappel Laboratories), diluted 1:1000 in assay buffer (0.5% bovine serum albumin, 0.05% Tween-20 in PBS), was added to each well and the plates incubated for 1 hour at room temperature on a shaker apparatus. The plates were washed three times with wash buffer, followed by addition of 50 µl of substrate (TMB Microwell Peroxidase Substrate; Kirkegaard & Perry, Gaithersburg, MD) to each well and incubation at room temperature for 10 minutes. The reaction was stopped by adding 50 µl of TMB 1-Component Stop Solution (Diethyl Glycol; Kirkegaard & Perry) to each well, and absorbance at 450 nm was read in an automated microtiter plate reader.

Hybridoma supernatants initially screened in the ELISA were considered for their ability to bind to DR4-IgG but not to CD4-IgG. The supernatants testing positive in the ELISA were further analyzed by FACS analysis using 9D cells (a human B lymphoid cell line expressing DR4; Genentech, Inc.) and FITC-conjugated goat anti-mouse IgG. For this analysis, 25 µl of cells suspended (at 4 X 10⁶ cells/ml) in cell sorter buffer (PBS containing 1% FCS and 0.02% NaN₃) were added to U-bottom microtiter wells, mixed with 100µl of culture supernatant or purified antibody (10µg/ml) in cell sorter buffer, and incubated for 30 minutes on ice. The cells were then washed and incubated with 100 µl FITC-conjugated goat anti-mouse IgG for 30 minutes at 4°C. Cells were then washed twice, resuspended in 150 µl of cell sorter buffer and then analyzed by FACScan (Becton Dickinson, Mountain View, CA).

The FACS staining of the 9D cells revealed that the antibodies, 4E7.24.3 and 4H6.17.8, recognized the DR4 receptor on the 9D cells. Hybridoma supernatants and purified antibodies were then tested for activity to induce DR4 mediated 9D cell apoptosis. The 9D cells (5 X 10⁵ cells/0.5ml) were incubated with 5 µg of DR4 mAbs (4E7.24.3 or 4H6.17.8) or IgG control antibodies in 200 µl complete RPMI media at 4°C for 15 minutes. The cells were then incubated for 5 minutes at 37°C with or without 10 µg of goat anti-mouse IgG Fc antibody (ICN Pharmaceuticals) in 300 µl of complete RPMI. At this point, the cells were incubated overnight at 37°C and in the presence of 7% CO₂. The cells were then harvested and washed once with PBS. The apoptosis of the cells was determined by staining of FITC-annexin V binding to phosphatidylserine according to manufacturer recommendations (Clontech). The cells were washed in PBS and resuspended in 200 µl binding buffer. Ten µl of annexin-V-FITC (1 µg/ml) and 10 µl of propidium iodide were added to the cells. After incubation for 15 minutes in the dark, the 9D cells were analyzed by FACS.
Both DR4 antibodies (in the absence of the goat anti-mouse IgG Fc) induced apoptosis in the 9D cells as compared to the control antibodies. Agonistic activity of both DR4 antibodies, however, was enhanced by DR4 receptor cross-linking in the presence of the goat anti-mouse IgG Fc. This enhanced apoptosis by both DR4 antibodies is comparable to the apoptotic activity of Apo-2L in 9D cells.

The *in vivo* study examining the effects of the 4H6.17.8 monoclonal antibody plus CPT-11 (as compared to other treatment groups indicated in Figure 2) was conducted essentially as described in Example 1 above, except that in the antibody treatment groups, anti-DR4 antibody 4H6 (5 mg/kg; prepared as described above) was administered by i.p. injection to the mice twice per week for the duration of the study. In the Apo-2L treatment groups, Apo-2L was administered by i.p. injection on days 0-4 at 60 mg/kg/day. In the CPT-11 treatment groups, CPT-11 was administered by i.v. injection on days 0, 4, and 8 at 80 mg/kg.

The results are shown in Figure 2. Each agent alone caused a significant delay in tumor progression. The combination of Apo-2L or anti-DR4 monoclonal antibody with CPT-11 caused tumor regression, with a much more delayed time to tumor progression as compared to the single agent treatments. The anti-DR4 monoclonal antibody was more effective than Apo-2L both as single agent and in combination with CPT-11. A partial response (tumor volume decreased by more than 50% of its initial value) occurred in all 10 mice treated with the anti-DR4 antibody plus CPT-11, but in only 6 out of 10 mice treated with the Apo-2L plus CPT-11. These results show that Apo-2L receptor agonists cooperate synergistically with CPT-11 to inhibit tumor progression beyond the additive sum of effects of the respective single agent treatments.

### Deposit of Material

The following materials have been deposited with the American Type Culture Collection, 10801 University Boulevard, Manassas, Virginia, USA (ATCC):

| | | |
|---|---|---|
| Material | ATCC Dep. No. | Deposit Date |
| 4E7.24.3 | HB-12454 | Jan. 13, 1998 |
| 4H6.17.8 | HB-12455 | Jan. 13, 1998 |
| 1H5.25.9 | HB-12695 | April 1, 1999 |
| 4G7.18.8 | PTA-99 | May 21, 1999 |
| 5G11.17.1 | HB-12694 | April 1, 1999 |
| 3F11.39.7 | HB-12456 | Jan. 13, 1998 |
| 3H3.14.5 | HB-12534 | June 2, 1998 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC Section 122 and the Commissioner's rules pursuant thereto (including 37 CFR Section 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written description is considered to be sufficient to enable one skilled in the art to practice the invention. The present invention is not to be limited in scope by the example presented herein. Indeed, various modifications of the invention in addition to those shown and described herein will become apparent to those skilled in the art from the foregoing description and fall within the scope of the appended claims.

## Claims

1. Use of agonistic anti-Apo-2 ligand receptor antibody in the manufacture of a medicament for the induction of apoptosis in mammalian cancer cells, wherein the medicament is for administration concurrently or sequentially with a synergistically effective amount of CPT-11.

2. Use of CPT-11 in the manufacture of a medicament for the induction of apoptosis in mammalian cancer cells, wherein the medicament is for administration concurrently or sequentially with a synergistically effective amount of agonistic anti-Apo-2 ligand receptor antibody.

3. Use of a synergistically effective amount of agonistic anti-Apo-2 ligand receptor antibody and CPT-11 in the manufacture of a medicament for the induction of apoptosis in mammalian cancer cells.

4. A method of inducing apoptosis in mammalian cancer cells in cell culture or ex vivo, comprising exposing mammalian cancer cells in cell culture or ex vivo to a synergistically effective amount of agonistic anti-Apo-2 ligand receptor antibody and CPT-11.

5. The use or method of any one of claims 1 to 4 wherein said agonistic antibody comprises an anti-DR4 receptor antibody.

6. The use or method of claim 5 wherein said anti-DR4 receptor antibody is a monoclonal antibody.

7. The use or method of claim 6 wherein said anti-DR4 receptor monoclonal antibody comprises a chimeric antibody.

8. The use or method of claim 6 wherein said anti-DR4 receptor monoclonal antibody comprises a human antibody.

9. The use or method of any one of claims 1 to 4 wherein said agonistic antibody comprises an anti-DRS receptor antibody.

10. The use or method of claim 9 wherein said anti-DR5 receptor antibody is a monoclonal antibody.

11. The use or method of claim 10 wherein said anti-DR5 receptor monoclonal antibody comprises a chimeric antibody.

12. The use or method of claim 10 wherein said anti-DR5 receptor monoclonal antibody comprises a human antibody.

13. The use or method of any one of claims 1 to 4 wherein said agonistic anti-Apo-2 ligand receptor antibody is an antibody which cross-reacts with more than one Apo-2 ligand receptor.

14. The use of any one of claims 1 to 3 wherein the medicament is for administration with one or more growth inhibitory agents.

15. The use of any one of claims 1 to 3 wherein the medicament is for administration with radiation therapy.

16. The method of claim 4, further comprising exposing the cancer cells to one or more growth inhibitory agents.

17. The method of claim 4, further comprising exposing the cancer cells to radiation.

18. The use or method of any one of claims 1 to 4 wherein the cancer cells comprise colorectal cancer cells.

19. A synergistically effective amount of agonistic anti-Apo-2 ligand receptor antibody and CPT-11 for concurrent or sequential administration in a method of medical treatment.

20. A composition comprising a synergistically effective amount of agonistic anti-Apo-2 ligand receptor antibody and CPT-11 in a carrier.

21. A kit comprising a synergistically effective amount of agonistic anti-Apo-2 ligand receptor antibody and CPT-11 for concurrent or sequential administration in the treatment of cancer in a mammal.

## Patentansprüche

1. Verwendung von agonistischem Anti-Apo-2-Ligandenrezeptor-Antikörper bei der Herstellung eines Medikaments zur Induktion von Apoptose in Säugetierkrebszellen, worin das Medikament zur Verabreichung gleichzeitig oder nacheinander mit einer synergistisch wirksamen Menge von CPT-11 konzipiert ist.

2. Verwendung von CPT-11 bei der Herstellung eines Medikaments zur Induktion von Apoptose in Säugetierkrebszellen, worin das Medikament zur Verabreichung gleichzeitig oder nacheinander mit einer synergistisch wirksamen Menge von agonistischem Anti-Apo-2-Ligandenrezeptor-Antikörper konzipiert ist.

3. Verwendung einer synergistisch wirksamen Menge von agonistischem Anti-Apo-2-Ligandenrezeptor-Antikörper und CPT-11 bei der Herstellung eines Medikaments zur Induktion von Apoptose in Säugetierkrebszellen.

4. Verfahren zur Induktion von Apoptose in Säugetierkrebszellen in Zellkultur oder ex vivo, umfassend das Aussetzen von Säugetierkrebszellen in Zellkultur oder ex vivo gegenüber einer synergistisch wirksamen Menge von agonistischem Anti-Apo-2-Ligandenrezeptor-Antikörper und CPT-11.

5. Verwendung oder Verfahren nach einem der Ansprüche 1 bis 4, worin der agonistische Antikörper einen Anti-DR4-Rezeptor-Antikörper umfasst.

6. Verwendung oder Verfahren nach Anspruch 5, worin der Anti-DR4-Rezeptor-Antikörper ein monoklonaler Antikörper ist.

7. Verwendung oder Verfahren nach Anspruch 6, worin der monoklonale Anti-DR4-Rezeptor-Antikörper einen chimären Antikörper umfasst.

8. Verwendung oder Verfahren nach Anspruch 6, worin der monoklonale Anti-DR4-Rezeptor-Antikörper einen menschlichen Antikörper umfasst.

9. Verwendung oder Verfahren nach einem der Ansprüche 1 bis 4, worin der agonistische Antikörper einen Anti-DR5-Rezeptor-Antikörper umfasst.

10. Verwendung oder Verfahren nach Anspruch 9, worin der Anti-DR5-Rezeptor-Antikörper ein monoklonaler Antikörper ist.

11. Verwendung oder Verfahren nach Anspruch 10, worin der monoklonale Anti-DR5-Rezeptor-Antikörper einen chimären Antikörper umfasst.

12. Verwendung oder Verfahren nach Anspruch 10, worin der monoklonale Anti-DR5-Rezeptor-Antikörper einen menschlichen Antikörper umfasst.

13. Verwendung oder Verfahren nach einem der Ansprüche 1 bis 4, worin der agonistische Anti-Apo-2-Ligandenrezeptor-Antikörper ein Antikörper ist, der mit mehr als einem Apo-2-Ligandenrezeptor wechselwirkt.

14. Verwendung nach einem der Ansprüche 1 bis 3, worin das Medikament zur Verabreichung mit einem oder mehreren Wachstumsinhibitoren konzipiert ist.

15. Verwendung nach einem der Ansprüche 1 bis 3, worin das Medikament zur Verabreichung mit Strahlentherapie konzipiert ist.

16. Verfahren nach Anspruch 4, weiters umfassend das Aussetzen der Krebszellen gegenüber einem oder mehreren Wachstumsinhibitoren.

17. Verfahren nach Anspruch 4, weiters umfassend das Aussetzen der Krebszellen gegenüber Bestrahlung.

18. Verwendung oder Verfahren nach einem der Ansprüche 1 bis 4, worin die Krebszellen Kolorektal-Krebszellen umfassen.

19. Synergistisch wirksame Menge von agonistischem Anti-Apo-2-Ligandenrezeptor-Antikörper und CPT-11 zur gleichzeitigen oder aufeinander folgenden Verabreichung in einem medizinischen Behandlungsverfahren.

20. Zusammensetzung, umfassend eine synergistisch wirksame Menge von agonistischem Anti-Apo-2-Ligandenrezeptor-Antikörper und CPT-11 in einem Träger.

21. Set, umfassend eine synergistisch wirksame Menge von agonistischem Anti-Apo-2-Ligandenrezeptor-Antikörper und CPT-11 zur gleichzeitigen oder aufeinander folgenden Verabreichung bei der Behandlung von Krebs in einem Säugetier.

## Revendications

1. Utilisation d'un anticorps agoniste anti-récepteur de ligand Apo-2 dans la fabrication d'un médicament pour l'induction d'apoptose dans des cellules cancéreuses de mammifère, dans laquelle le médicament est destiné à une administration simultanée ou successive avec une quantité synergistiquement efficace de CPT-11.

2. Utilisation de CPT-11 dans la fabrication d'un médicament pour l'induction d'apoptose dans des cellules cancéreuses de mammifère, dans laquelle le médicament est destiné à une administration simultanée ou successive avec une quantité synergistiquement efficace d'anticorps agoniste anti-récepteur de ligand Apo-2.

3. Utilisation d'une quantité synergistiquement efficace d'anticorps agoniste anti-récepteur de ligand Apo-2 et de CPT-11 dans la fabrication d'un médicament pour l'induction d'apoptose dans des cellules cancéreuses de mammifère.

4. Procédé d'induction d'apoptose dans des cellules cancéreuses de mammifère en culture cellulaire ou ex vivo, comprenant l'exposition des cellules cancéreuses de mammifère en culture cellulaire ou ex vivo à une quantité synergistiquement efficace d'anticorps agoniste anti-récepteur de ligand Apo-2 et de CPT-11.

5. Utilisation ou procédé selon l'une quelconque des revendications 1 à 4, dans lequel(laquelle) ledit anticorps agoniste comprend un anticorps anti-récepteur DR4.

6. Utilisation ou procédé selon la revendication 5, dans lequel(laquelle) ledit anticorps anti-récepteur DR4 est un anticorps monoclonal.

7. Utilisation ou procédé selon la revendication 6, dans lequel(laquelle) ledit anticorps monoclonal anti-récepteur DR4 comprend un anticorps chimérique.

8. Utilisation ou procédé selon la revendication 6, dans lequel(laquelle) ledit anticorps monoclonal anti-récepteur DR4 comprend un anticorps humain.

9. Utilisation ou procédé selon l'une quelconque des revendications 1 à 4, dans lequel(laquelle) ledit anticorps agoniste comprend un anticorps anti-récepteur DR5.

10. Utilisation ou procédé selon la revendication 9, dans lequel(laquelle) ledit anticorps anti-récepteur DR5 est un anticorps monoclonal.

11. Utilisation ou procédé selon la revendication 10, dans lequel(laquelle) ledit anticorps monoclonal anti-récepteur DR5 comprend un anticorps chimérique.

12. Utilisation ou procédé selon la revendication 10, dans lequel(laquelle) ledit anticorps monoclonal anti-récepteur DR5 comprend un anticorps humain.

13. Utilisation ou procédé selon l'une quelconque des revendications 1 à 4, dans lequel(laquelle) ledit anticorps agoniste anti-récepteur de ligand Apo-2 est un anticorps qui réagit de manière croisée avec plus d'un récepteur de ligand Apo-2.

14. Utilisation selon l'une quelconque des revendications 1 à 3, dans laquelle le médicament est destiné à une administration avec un ou plusieurs agents inhibiteurs de croissance.

15. Utilisation selon l'une quelconque des revendications 1 à 3, dans laquelle le médicament est destiné à une administration avec une radiothérapie.

16. Procédé selon la revendication 4, comprenant en outre l'exposition des cellules cancéreuses à un ou plusieurs agents inhibiteurs de croissance.

17. Procédé selon la revendication 4, comprenant en outre l'exposition des cellules cancéreuses à une radiation.

18. Utilisation ou procédé selon l'une quelconque des revendications 1 à 4, dans lequel(laquelle) les cellules cancéreuses comprennent des cellules d'adénocarcinome colorectal.

19. Quantité synergistiquement efficace d'anticorps agoniste anti-récepteur de ligand Apo-2 et de CPT-11 destinée à une administration simultanée ou successive dans un procédé de traitement médical.

20. Composition comprenant une quantité synergistiquement efficace d'anticorps agoniste anti-récepteur de ligand Apo-2 et de CPT-11 dans un support.

21. Trousse comprenant une quantité synergistiquement efficace d'anticorps agoniste anti-récepteur de ligand Apo-2 et de CPT-11 destinée à une administration simultanée ou successive dans le traitement d'un cancer chez un mammifère.
